# FASCICULE DE BREVET EUROPEEN

(11) **EP 2 703 091 B1**
(45) Date de publication et mention de la délivrance du brevet: **20.07.2016**
(21) Numéro de dépôt: 13182091.2
(22) Date de dépôt: 28.08.2013
(51) Int. Cl.: B05B 11/00, A61M 15/00, A61M 11/08, A61M 15/08

(54) **Flacon de distribution d'un produit fluide**
Sprühfläschchen für ein flüssiges Produkt
Dispensing bottle for a fluid product

(30) Priorité: 04.09.2012 FR 1258251
(43) Date de publication de la demande: 05.03.2014
(73) Titulaire: Albéa le Tréport, 76470 Le Tréport (FR)
(72) Inventeur: Dumont, Pierre, 80570 Dargnies (FR); Elmeguenni, Mohamed, 80350 Mers les Bains (FR); Lasnier, Jacky, 76480 Sainte Marguerite sur Duclair (FR); Maudit, Emmanuel, 80100 Abbeville (FR); Octau, Jean-Luc, 76630 Intraville (FR)
(74) Mandataire: Gevers & Orès

(56) Documents cités:
- EP-A1- 0 657 223
- EP-A1- 2 441 344
- EP-A1- 2 532 443
- WO-A1-03/066235
- WO-A2-2006/129044

## Description

L'invention concerne un flacon de distribution d'un produit fluide, notamment liquide, par exemple d'un produit cosmétique de soin, de maquillage ou de parfumage, ou d'un produit pharmaceutique.

Le flacon de distribution comprend un corps dans lequel un réservoir de conditionnement du produit est formé, ainsi qu'un dispositif de prélèvement du produit conditionné qui est monté de façon étanche dans ledit corps. En particulier, le dispositif de prélèvement peut comprendre une pompe à actionnement manuel qui est alimentée en produit conditionné, ladite pompe étant agencée pour distribuer le produit sous pression, par exemple sous la forme d'un aérosol.

Pour ce faire, le dispositif de prélèvement est équipé d'un bouton poussoir qui est pourvu d'un orifice de distribution du produit, ledit bouton poussoir étant déplaçable réversiblement sur une course d'actionnement dudit dispositif entre une position au repos et une position enfoncée dans laquelle l'orifice de distribution est en communication avec le réservoir par l'intermédiaire dudit dispositif.

Dans un exemple d'application, les flacons selon l'invention permettent la distribution d'échantillons de produit, notamment pour un volume de produit conditionné dans le réservoir qui est compris entre 1 et 10 ml. En particulier, les échantillons ainsi distribués peuvent permettre à un client de tester le produit, les flacons étant alors qualifiés de flacons testeurs d'échantillons. En variante, les flacons peuvent être dits « de sac » en ce qu'ils permettent de transporter facilement un volume réduit de produit, par opposition à des flacons de contenance supérieure qui sont en général lourds et encombrants car cossus.

Dans ces applications, par exemple pour des raisons logistiques, de praticité ou encore environnementales de recyclage, il peut être souhaitable de pouvoir recharger le réservoir en produit à partir d'une source dudit produit. En effet, il est peu pratique pour un utilisateur d'effectuer le remplissage du réservoir à l'aide d'un petit entonnoir et peu écologique de jeter un flacon vide pour le remplacer par un plein constituant recharge.

Des flacons de distribution sont déjà proposés à la vente, dans lesquels le corps est équipé d'une soupape de remplissage du réservoir qui est agencée pour permettre la mise en communication d'une source de produit avec ledit réservoir. En particulier, la soupape peut s'ouvrir par appui sur le tube de sortie de la pompe d'un flacon source qu'il convient d'actionner à plusieurs reprises pour réaliser le remplissage du réservoir par injection du produit source, ce qui est un geste peu intuitif pour l'utilisateur.

Le document FR-2 854 131 prévoit le vidage d'un réservoir au moyen d'une pompe de distribution sans reprise d'air dans le réservoir de conditionnement en compensation du volume de produit distribué de sorte à créer une dépression d'air dans ledit réservoir, ladite dépression permettant de réaliser le remplissage dudit réservoir par aspiration du produit source.

Le document EP-2 441 344 A1, qui décrit un flacon de distribution selon le préambule de la revendication 1, propose des flacons de distribution dont le réservoir est vide de produit et présente une dépression d'air qui est agencée pour pouvoir réaliser ultérieurement le remplissage initial du réservoir en produit par mise en communication étanche d'une source de produit avec ledit réservoir par l'intermédiaire d'une soupape de sorte que ladite dépression induise le remplissage dudit réservoir par aspiration du produit contenu dans ladite source.
Toutefois, dans les solutions de remplissage par aspiration, se pose le problème de la conservation dans le temps de la dépression d'air dans le réservoir. En effet, les dispositifs de prélèvement ne sont jamais parfaitement étanches aux micro-fuites d'air car ils comprennent de nombreuses zones d'étanchéité par serrage et sont composés de matières plastiques ou élastomériques qui, dans le temps, s'avèrent légèrement poreuses à l'air. L'invention vise à perfectionner l'art antérieur en proposant notamment un flacon de distribution dans lequel une dépression d'air dans le réservoir peut être conservée de façon prolongée dans le temps, notamment lors du stockage du flacon avant son remplissage initial, de sorte à fiabiliser la capacité de remplissage par aspiration dudit réservoir par mise en communication étanche d'une source de produit avec ledit réservoir.

A cet effet, l'invention propose un flacon de distribution d'un produit fluide comprenant un corps dans lequel un réservoir destiné au conditionnement dudit produit est formé, ledit flacon comprenant en outre un dispositif de prélèvement dudit produit conditionné qui est monté de façon étanche dans ledit corps, ledit dispositif de prélèvement étant équipé d'un bouton poussoir qui est pourvu d'un orifice de distribution dudit produit, ledit bouton poussoir étant déplaçable réversiblement sur une course d'actionnement dudit dispositif entre une position au repos et une position enfoncée dans laquelle l'orifice de distribution est en communication avec le réservoir par l'intermédiaire dudit dispositif, ledit corps étant équipé d'une soupape de remplissage dudit réservoir qui est agencée pour permettre la mise en communication d'une source de produit avec ledit réservoir, le réservoir présentant une dépression d'air qui est agencée pour pouvoir réaliser le remplissage du réservoir en produit par aspiration du produit contenu dans ladite source, une paroi supérieure étant intégrée au corps par l'intermédiaire d'un moyen de liaison étanche qui peut être détruit manuellement, ledit dispositif de prélèvement étant monté dans ledit corps avec le bouton poussoir maintenu en position enfoncée sur ladite paroi supérieure de sorte à mettre ledit moyen de liaison en communication avec ledit réservoir.

D'autres objets et avantages de l'invention apparaîtront dans la description qui suit, faite en référence aux figures annexées, dans lesquelles :
- la figure 1 est une vue en coupe longitudinale d'un flacon de distribution selon l'invention qui est représenté en état de stockage, les figures 1a à 1c étant des vues agrandies des zones respectivement A, B et C de la figure 1 ;
- la figure 2 est une vue en coupe longitudinale du flacon de la figure 1 montrant le remplissage du réservoir au moyen d'une source de produit, la figure 2a étant une vue agrandie de la zone A de la figure 2 ;
- la figure 3 est une vue en coupe longitudinale du flacon de la figure 1 qui est représenté en état d'utilisation ;
- les figures 4 à 6 sont des vues des composants de la soupape de remplissage du flacon de la figure 1, montrant respectivement le chapeau en perspective de dessous (figure 4), le clapet déformable en coupe longitudinale (figure 5) et l'armature du clapet déformable en perspective de dessus (figure 6).

Dans la description, les termes de positionnement dans l'espace sont pris en référence à la position du flacon représenté sur les figures.

En relation avec les figures, on décrit ci-dessous un flacon destiné à contenir un produit fluide en vue de sa distribution. Dans des exemples particuliers, le produit peut être liquide, notamment un produit cosmétique de soin, de maquillage ou de parfumage, ou un produit pharmaceutique.

Le flacon comprend un corps 1 dans lequel un réservoir 2 de conditionnement du produit est formé. Selon une application particulière, le réservoir 2 peut avoir une contenance comprise entre 1 et 10 ml de sorte à permettre la distribution d'échantillons de produit.

Dans les modes de réalisation représentés, le corps 1 est rigide, notamment en présentant une rigidité suffisante pour que le volume du réservoir 2 demeure sensiblement constant, même si la pression interne varie. Le corps 1 peut être monobloc, par exemple réalisé par injection-soufflage ou extrusion-soufflage, ou en plusieurs parties injectées puis assemblées, par exemple par soudure ultra-sons, ou par laser, ou par friction rotative, en matière plastique rigide, en métal, par exemple en aluminium, ou en verre.

Le flacon comprend un dispositif de prélèvement du produit conditionné qui est monté de façon étanche dans le corps 1. En particulier, le dispositif de prélèvement est équipé d'un bouton poussoir 3 qui est pourvu d'un orifice de distribution 4 dudit produit, ledit bouton poussoir étant déplaçable réversiblement sur une course d'actionnement dudit dispositif entre une position au repos et une position enfoncée dans laquelle l'orifice de distribution 4 est en communication avec le réservoir 2 par l'intermédiaire dudit dispositif.

Dans le mode de réalisation représenté, le dispositif de prélèvement comprend une pompe 5 de distribution actionnée manuellement au moyen d'un bouton poussoir 3 qui est alimenté avec le produit sous pression en vue de sa distribution.

La pompe 5 comprend des moyens d'alimentation en produit conditionné qui, sur les figures, comprennent un tube plongeur 6 disposé dans le réservoir 2, ledit tube étant équipé d'un clapet 7 d'entrée du produit dans la pompe 5. Le bouton poussoir 3 est monté sur le gicleur 8 de la pompe 3 en mettant l'orifice de distribution 4 en communication avec un canal de sortie 9 dudit gicleur.

La pompe 5 comprend également un carter 10 dans lequel un piston 11 monté autour du gicleur 8 est disposé pour délimiter une chambre de dosage 12 dans ledit carter, ledit gicleur étant déplaçable réversiblement sur une course de distribution - respectivement d'aspiration - dans laquelle le piston 11 ouvre - respectivement ferme - la communication entre le canal de sortie 9 et la chambre de dosage 12.

Le bouton poussoir 3 comprend une zone supérieure 3a permettant à l'utilisateur d'exercer un appui digital sur ledit bouton poussoir afin de pouvoir déplacer le gicleur 8 sur sa course de distribution jusqu'à une position enfoncée dudit bouton poussoir, le retour du bouton poussoir 3 en position de repos sur la course d'aspiration du gicleur 8 étant classiquement réalisé par un ressort 13.

Dans le mode de réalisation représenté, le bouton poussoir 3 est équipé d'une buse de pulvérisation 14 qui est agencée pour distribuer radialement un aérosol du produit au travers de l'orifice de distribution 4. Toutefois, l'invention n'est pas limitée à un mode particulier de distribution du produit. En particulier, notamment pour un embout nasal de pulvérisation, le bouton poussoir 3 peut permettre une distribution axiale du produit et un autre type de dispositif de prélèvement peut être envisagé.

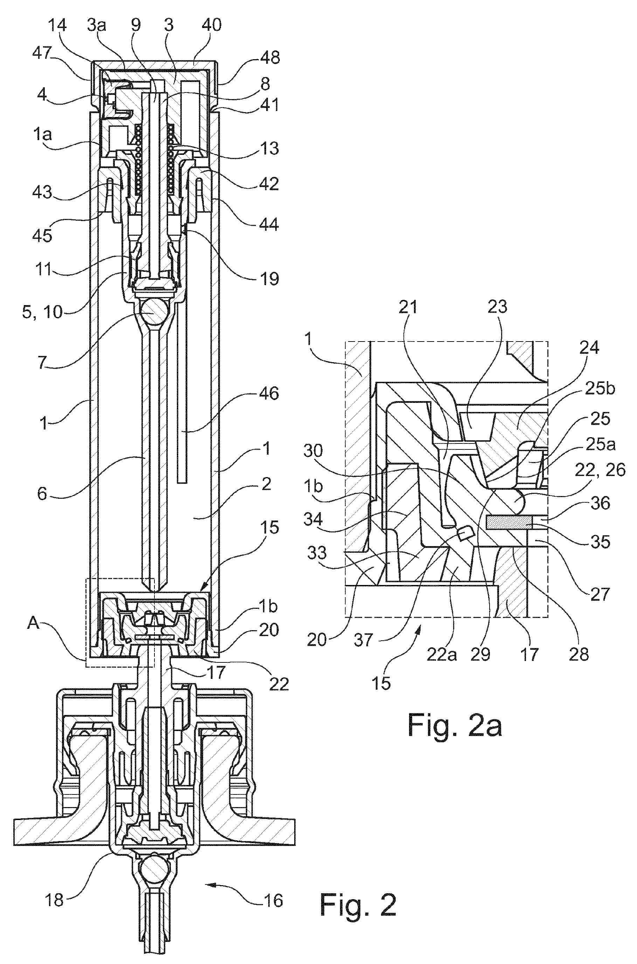

Le corps 1 du flacon est équipé d'une soupape 15 de remplissage du réservoir 2 qui est agencée pour permettre la mise en communication d'une source de produit 16 avec ledit réservoir. En relation avec la figure 2, la source de produit 16 comprend un réservoir source sur lequel est disposé un tube de sortie 17, le remplissage en produit du réservoir 2 étant réalisé par montage dudit tube en appui étanche sur la soupape 15 qui est agencée pour s'ouvrir de façon réversible.

En particulier, on peut utiliser en tant que source de produit 16 un flacon nourrice de contenance supérieure, ledit flacon étant équipé d'une pompe 18 dont le bouton poussoir est retiré pour permettre la disposition du gicleur 17 en appui étanche sur la soupape 15. En effet, outre l'ouverture de la soupape 15, l'appui étanche provoque l'ouverture de la pompe 18 afin de permettre le passage du produit de remplissage au travers d'elle.

Selon une autre réalisation, le réservoir source 16 est formé à l'intérieur d'une poche souple qui peut être remplie de produit sans air ni gaz pour la bonne conservation dudit produit. Le transfert du produit dans le réservoir est alors possible dans toutes les positions et la poche souple ne peut pas être détournée de son rôle de source puisque sans gaz propulseur ni pression interne, ni bouton poussoir pour actionner une éventuelle pompe ou valve associée au tube de sortie 17.

Le réservoir 2 présente une dépression d'air qui est agencée pour pouvoir réaliser le remplissage dudit réservoir en produit par mise en communication étanche de la source de produit 16 avec ledit réservoir par l'intermédiaire de la soupape 15 de sorte que ladite dépression induise le remplissage dudit réservoir par aspiration du produit contenu dans ladite source. En particulier, la dépression d'air peut être de l'ordre de - 980 hPa.

Le réservoir 2 peut être vide de produit et présenter une dépression d'air initiale qui est utilisée pour réaliser le premier remplissage dudit réservoir en produit. Le dispositif de prélèvement 5 peut alors être du type sans reprise d'air pour permettre des remplissages ultérieurs ou avec reprise d'air pour limiter l'utilisation du flacon à un seul remplissage. Dans le mode de réalisation représenté, la pompe 5 est un modèle avec reprise d'air et présente donc un trou d'évent 19 qui est agencé pour permettre de compenser le volume de produit prélevé dans le réservoir 2 par de l'air.

En particulier, la dépression d'air initiale peut être réalisée par mise en communication du réservoir 2 vide de produit avec un dispositif d'aspiration d'air. Pour ce faire, on peut par exemple utiliser un dispositif d'aspiration d'air qui comprend une cloche à vide dans laquelle le flacon de distribution est disposé, le montage étanche du dispositif de prélèvement 5 dans le corps 1 étant réalisé après activation de ladite cloche. Ainsi, la dépression est formée dans le réservoir 2 puis le dispositif de prélèvement 5 est monté de façon étanche pour maintenir ladite dépression.

On peut également utiliser un dispositif d'aspiration d'air comprenant une pompe à vide, ledit dispositif d'aspiration étant mis en communication étanche avec la soupape 15 pour, après le montage étanche du dispositif de prélèvement 5 dans le corps 1, aspirer l'air du réservoir 2 au travers de ladite soupape.

Dans le mode de réalisation représenté, la soupape 15 comprend un chapeau rigide 20 présentant un logement interne 21 dans lequel un clapet déformable 22 est monté, ledit chapeau étant monté de façon étanche dans le corps 1 pour mettre le logement interne 21 en communication avec le réservoir 2 par l'intermédiaire d'un passage aval 23. En variante, la soupape 15 peut comprendre un clapet déplaçable entre un état stable de fermeture du passage aval 23 et un état contraint d'ouverture dudit passage aval.

Le logement interne 21 présente une structure 24 équipée d'un passage intermédiaire 25 formé entre une ouverture amont 25a et une ouverture aval 25b, le clapet déformable 22 présentant un siège 26 qui est équipé d'un passage amont 27 formé entre une portée inférieure 28 de réception du tube de sortie 17 de la source de produit 16 et une portée supérieure 29 qui est surmontée par une lèvre d'étanchéité 30.

En outre, la soupape 15 est agencée pour présenter un état stable, dans lequel la lèvre d'étanchéité 30 occulte de façon étanche l'ouverture aval 25b, et un état contraint par appui axial du tube de sortie 17 sur la portée inférieure 29, dans lequel le siège 26 est comprimé par écrasement axial de la portée supérieure 29 sur la structure 24 en permettant la communication entre le passage amont 27 et l'ouverture amont 25a, ladite compression induisant un décollement de ladite lèvre pour ouvrir la communication entre l'ouverture aval 25b et le passage aval 23.

Ainsi, le remplissage du réservoir 2 est réalisé par simple appui du tube de sortie 17 pour mettre en communication étanche ledit tube de sortie avec le réservoir 2 par l'intermédiaire des passages 27, 25, 23, le retrait dudit tube de sortie induisant le retour de la soupape 15 en état stable en fiabilisant l'étanchéité au travers d'elle par occultation de l'ouverture aval 25b.

De façon avantageuse, l'étanchéité est fiabilisée en prévoyant que la lèvre d'étanchéité 30 soit reliée au siège 26 de sorte que la compression - respectivement l'extension - axiale dudit siège entre ses portées supérieure 29 et inférieure 28 induise un décollement - respectivement un plaquage - radial de ladite lèvre sur l'ouverture aval 25b.

En particulier, les portées inférieure 28 et supérieure 29 sont annulaires et s'étendent radialement autour du passage amont 27 en étant espacées axialement, la lèvre 30 étant également annulaire et s'étendant radialement autour de ladite portée supérieure en étant reliée à elle par l'intermédiaire d'un pan 31 qui est incliné vers l'extérieur. Ainsi, lorsque la portée supérieure 29 est comprimée sur la structure 24, l'inclinaison du pan 31 facilite le décollement radial de la lèvre 30.

Par ailleurs, la dépression d'air formée dans le réservoir 2 tend au plaquage étanche de la lèvre d'étanchéité 30 sur l'ouverture aval 25b afin de fiabiliser la conservation dans le temps de ladite dépression. En outre, pour améliorer l'étanchéité de la soupape 15, empêcher sa pollution par des corps étrangers venant de l'extérieur et constituer un témoin de première utilisation, ladite soupape peut être recouverte de façon réversible par un opercule 32 d'étanchéité que l'utilisateur retire ou déchire lorsqu'il souhaite remplir le réservoir 2. En particulier, l'opercule 32 peut être soudé sous le chapeau 20, ledit chapeau étant lui-même soudé dans le corps 1 pour assurer l'étanchéité du montage de la soupape 15 dans ledit corps.

De façon avantageuse, le clapet 22 peut comprendre une armature 33 sur laquelle est associé, notamment par surmoulage pour faciliter l'assemblage de la soupape 15, un élément 22a en matériau élastiquement déformable. Le siège 26 est formé sur l'élément 22a, l'armature 33 comprenant une paroi périphérique 34 d'association au chapeau 20 et un plateau 35 disposé entre les portées supérieure 29 et inférieure 28 en présentant un orifice 36 par lequel le passage amont 27 s'étend.

En particulier, le plateau 35 s'étend radialement et est relié à la paroi périphérique 34 par au moins un bras 37 permettant un déplacement élastique axial dudit plateau à l'intérieur de ladite paroi périphérique. Ainsi, en état stable, les bras 37 peuvent exercer un effort de plaquage de la lèvre d'étanchéité 30 sur l'ouverture aval 25b afin de fiabiliser l'étanchéité conférée.

De plus, le plateau 35 assure la répartition uniforme de l'effort exercé par le tube de sortie 17 lors de l'opération de remplissage, quelque soient le diamètre et le centrage dudit tube.

Dans le mode de réalisation représenté, la structure comprend un plot 24 présentant une paroi périphérique délimitée entre une surface intérieure 24a, une surface extérieure 24b et un bord inférieur libre 24c, le passage intermédiaire 25 comprenant au moins un conduit formé en creux dans ladite paroi avec l'ouverture amont 25a - respectivement aval 25b, débouchant dans la surface intérieure 24a - respectivement extérieure 24b.

En particulier, le passage amont 25a de chaque conduit 25 débouche dans le plot de la structure 24, la portée supérieure 29 étant disposée en regard du bord inférieur 24c pour être écrasée sur lui en état contraint et la lèvre d'étanchéité 30 à l'état stable est en appui étanche sur la surface extérieure 24b.

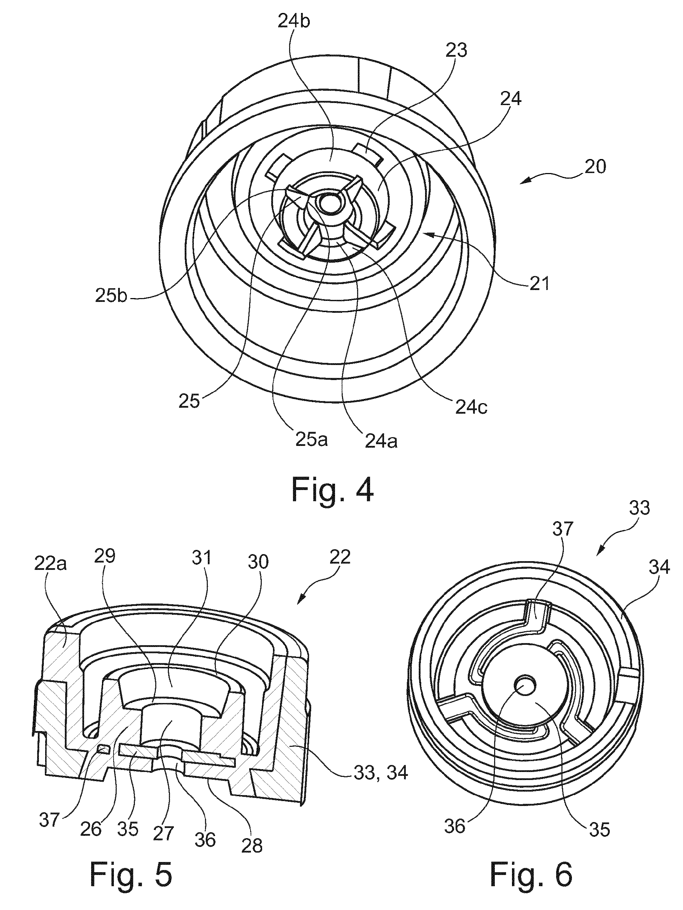

En relation avec la figure 4, le passage intermédiaire comprend quatre conduits 25 espacés angulairement d'environ 90°, lesdits conduits débouchant dans le bord inférieur 24c du plot 24. En outre, les conduits 25 sont inclinés vers le bas entre leur ouverture amont 25a et aval 25b, ladite ouverture aval étant occultée par la base du pan 31 afin de fiabiliser l'étanchéité conférée. Par ailleurs, le passage aval 23 comprend quatre orifices qui sont disposés en regard de respectivement un conduit 25.

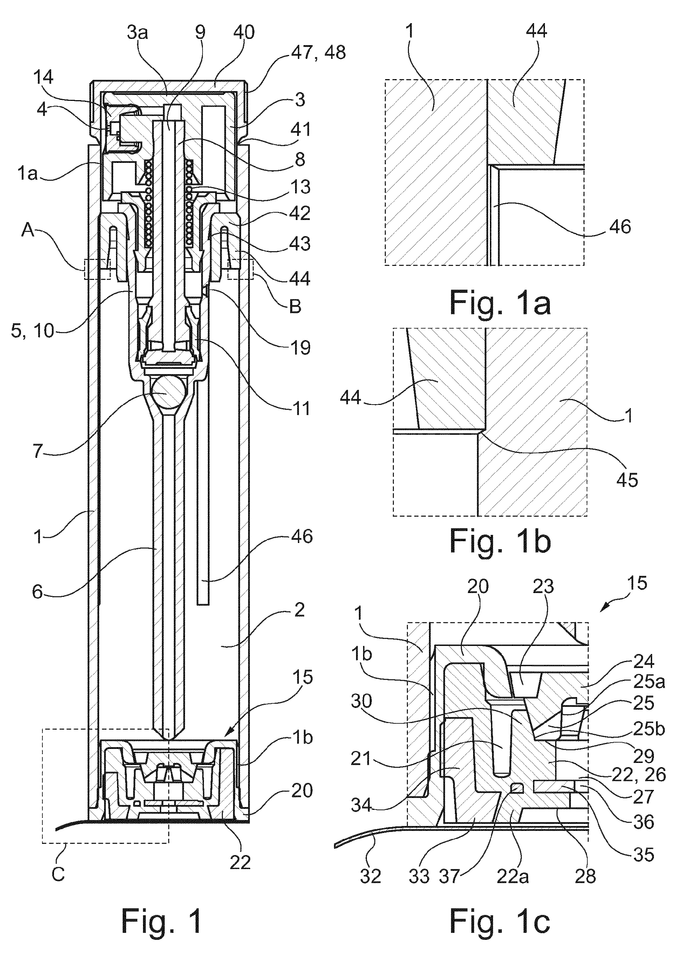

En relation avec les figures 1 et 2, une paroi supérieure 40 est intégrée au corps 1 par l'intermédiaire d'un moyen de liaison étanche 41 qui peut être détruit manuellement, le dispositif de prélèvement 5 étant monté dans ledit corps avec le bouton poussoir 3 maintenu en position enfoncée sur ladite paroi supérieure de sorte à mettre ledit moyen de liaison en communication avec le réservoir 2. De façon avantageuse, le maintien en position enfoncée est réalisé par mise en appui axial de la zone supérieure 3a sous la paroi supérieure 40.

Ainsi, lors du stockage du flacon, l'étanchéité de la dépression d'air du réservoir 2 n'est pas réalisée au niveau du dispositif de prélèvement 5 qui est maintenu en position ouverte, mais par l'intégration de la paroi supérieure 40 au corps 1, ladite intégration pouvant être facilement agencée pour conserver dans le temps ladite dépression d'air, notamment sans nécessiter de modification du dispositif de prélèvement 5.

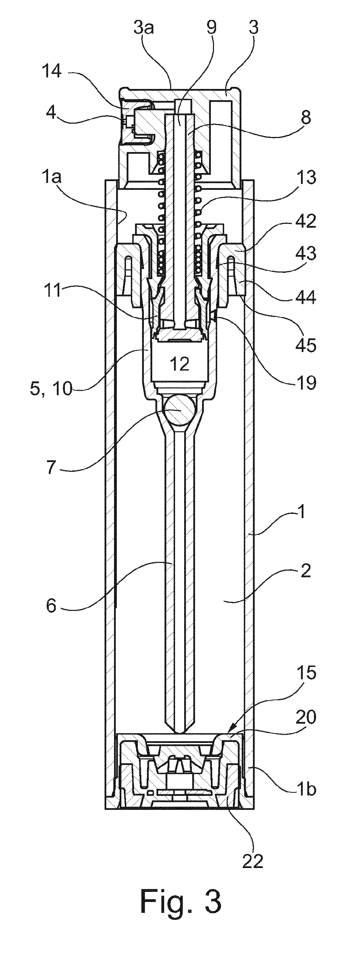

Ensuite, l'utilisateur peut remplir le réservoir 2 par aspiration (figures 2) puis rompre le moyen de liaison 41 pour libérer le bouton poussoir 3 qui se place alors en position de repos qui correspond à l'état d'utilisation du flacon (figure 3). En variante, le moyen de liaison 41 peut être rompu avant le remplissage du réservoir 2.

Dans le mode de réalisation représenté, le corps 1 présente une ouverture supérieure 1 a qui est occultée par la paroi supérieure 40 et une ouverture inférieure 1 b dans laquelle le chapeau 20 de la soupape de remplissage 15 est monté de façon étanche, de sorte notamment à remplir le réservoir 2 par le fond du flacon, ce qui correspond à un geste intuitif.

En outre, le dispositif de prélèvement 5 est monté dans le corps 1 par l'intermédiaire d'une douille 42, ladite douille comprenant un alésage 43 dans lequel le carter 10 est fixé et une couronne extérieure 44 en appui étanche sur l'intérieur dudit corps. En particulier, le dispositif de prélèvement 5 équipé de la douille 42 est introduit dans l'ouverture inférieure 1 b puis translaté dans le corps 1 jusqu'à mise du bouton poussoir 3 en position enfoncée par appui de la zone supérieure 3a sous la paroi supérieure 40.

Pour fiabiliser le maintien du bouton poussoir 3 en position enfoncée, l'intérieur du corps 1 présente une rainure radiale 45 sur laquelle la couronne extérieure 44 vient en appui axial à la fin du montage du dispositif de prélèvement 5 dans ledit corps (figure 1 b).

Par ailleurs, l'intérieur du corps 1 présente au moins une rainure axiale 46 d'éventation permettant un échappement d'air lors du montage par coulissement de la douille 42 dans le corps 1 (figure 1a). En outre, pour faciliter l'échappement d'air, l'intérieur du corps 1 peut présenter une géométrie conique qui converge vers le haut.

Dans le mode de réalisation représenté, le corps comprend une zone amincie 41 qui est disposée sous la paroi supérieure 40 pour former le moyen de liaison étanche. En particulier, la zone amincie 41 s'étend sur toute la périphérie de l'ouverture supérieure 1a du corps 1 afin de pouvoir séparer la paroi supérieure 40 dudit corps après destruction de ladite zone.

Par ailleurs, la paroi supérieure 40 est entourée d'une jupe 47, la zone amincie 41 du moyen de liaison étanche étant formée sous ladite jupe. En relation avec les figures, la paroi supérieure 40 et la jupe 47 forment ainsi un capot qui, lorsqu'il est relié au corps 1 par le moyen de liaison étanche 41, délimite un espace interne dans lequel est disposé le bouton poussoir 3 en position enfoncée.

En particulier, l'espace interne est en communication avec le réservoir 2 puisque, en position enfoncée du bouton poussoir 3, le canal de sortie 9 du gicleur 8 et donc l'orifice de distribution 4 débouchant dans ledit espace interne est en communication avec le réservoir 2 par l'intermédiaire de la chambre de dosage 14. En outre, l'espace interne est en communication avec le réservoir 2 par l'intermédiaire du trou d'évent 19.

Par ailleurs, pour inciter l'utilisateur à saisir la jupe 47 pour détruire le moyen de liaison étanche 41, ladite jupe peut présenter une portée extérieure pourvue de stries 48, lesdites stries permettant en outre de faciliter la préhension de ladite jupe par ledit utilisateur pour la faire tourner et/ou la soulever, de sorte à briser la zone amincie 41 pour libérer le bouton poussoir 3.

## Revendications

1. Flacon de distribution d'un produit fluide comprenant un corps (1) dans lequel un réservoir (2) destiné au conditionnement dudit produit est formé, ledit flacon comprenant en outre un dispositif de prélèvement (5) dudit produit conditionné qui est monté de façon étanche dans ledit corps, ledit dispositif de prélèvement étant équipé d'un bouton poussoir (3) qui est pourvu d'un orifice de distribution (4) dudit produit, ledit bouton poussoir étant déplaçable réversiblement sur une course d'actionnement dudit dispositif entre une position au repos et une position enfoncée dans laquelle l'orifice de distribution (4) est en communication avec le réservoir (2) par l'intermédiaire dudit dispositif de prélèvement (5), ledit
corps étant équipé d'une soupape de remplissage (15) dudit réservoir qui est agencée pour permettre la mise en communication d'une source de produit (16) avec ledit réservoir, le réservoir (2) présentant une dépression d'air qui est agencée pour pouvoir réaliser le remplissage du réservoir (2) en produit par aspiration du produit contenu dans ladite source, ledit flacon étant **caractérisé en ce qu'**une paroi supérieure (40) est intégrée au corps (1) par l'intermédiaire d'un moyen de liaison étanche (41) qui peut être détruit manuellement, ledit dispositif de prélèvement (5)
étant monté dans ledit corps avec le bouton poussoir (3) maintenu en position enfoncée sur ladite paroi supérieure (40) de sorte à mettre
ledit moyen de liaison étanche (41) en communication avec ledit réservoir.

2. Flacon de distribution selon la revendication 1, **caractérisé en ce que** le corps (1) comprend une zone amincie (41) qui est disposée sous la paroi supérieure (40) pour former le moyen de liaison étanche.

3. Flacon de distribution selon la revendication 1 ou 2, **caractérisé en ce que** la paroi supérieure (40) est entourée d'une jupe (47), le moyen de liaison étanche (41) étant formé sous ladite jupe.

4. Flacon de distribution selon la revendication 3, **caractérisé en ce que** la jupe (47) présente une portée extérieure pourvue de stries (48).

5. Flacon de distribution selon l'une quelconque des revendications 1 à 4, **caractérisé en ce que** le moyen de liaison étanche (41) s'étend sur toute la périphérie du corps (1) afin de pouvoir séparer la paroi supérieure (40) dudit corps après destruction dudit moyen de liaison.

6. Flacon de distribution selon l'une quelconque des revendications 1 à 5, **caractérisé en ce que** le corps (1) présente une ouverture inférieure (1b) dans laquelle la soupape de remplissage (15) est montée de façon étanche.

7. Flacon de distribution selon l'une quelconque des revendications 1 à 6, **caractérisé en ce que** le dispositif de prélèvement (5) présente un carter (10) qui est monté dans le corps (1) par l'intermédiaire d'une douille (42), ladite douille comprenant un alésage (43) dans lequel ledit carter est fixé et une couronne extérieure (44) en appui étanche sur l'intérieur dudit corps.

8. Flacon de distribution selon la revendication 7, **caractérisé en ce que** l'intérieur du corps (1) présente une rainure radiale (45) sur laquelle la couronne extérieure (44) est en butée axiale.

9. Flacon de distribution selon la revendication 7 ou 8, **caractérisé en ce que** l'intérieur du corps (1) présente au moins une rainure axiale (46) d'éventation lors du montage de la douille (42) dans le corps (1).

10. Flacon de distribution selon l'une quelconque des revendications 1 à 9, **caractérisé en ce que** l'intérieur du corps (1) présente une géométrie conique qui converge vers le haut.

11. Flacon de distribution selon l'une quelconque des revendications 1 à 10, **caractérisé en ce que** la soupape (15) comprend un chapeau rigide (20) présentant un logement interne (21) dans lequel un clapet déformable (22) est monté, ledit chapeau étant monté de façon étanche dans ledit corps pour mettre le logement interne (21) en communication avec le réservoir (2) par l'intermédiaire d'un passage aval (23).

## Patentansprüche

1. Abgabeflakon eines flüssigen Produkts, umfassend einen Körper (1), in welchem ein Reservoir (2) zur Verpackung des besagten Produkts gebildet ist, wo besagter Flakon darüber hinaus eine Entnahmevorrichtung (5) des besagten, verpackten Produkts umfasst, die dicht im besagten Körper montiert ist, wobei die besagte Entnahmevorrichtung mit einem Druckknopf (3) bestückt ist, der eine Verteiler-Öffnung (4) des besagten Produkts aufweist, wobei der besagte Druckknopf umkehrbar über einen Aktivierungsweg der besagten Vorrichtung zwischen einer Ruheposition und einer eingedrückten Position bewegt werden kann, wobei die Verteiler-Öffnung (4) in der eingedrückten Position über die besagte Entnahmevorrichtung (5) mit dem Reservoir (2) kommuniziert, wobei der besagte Körper mit einem Füllventil (15) des besagten Reservoirs ausgestattet ist, welches so angeordnet ist, dass es die Kommunikation zwischen einer Produktquelle (16) und dem besagten Reservoir etabliert, wobei das Reservoir (2) einen Luft-Unterdruck aufweist, der deshalb beaufschlagt wird, um das Auffüllen des Reservoirs (2) mit dem Produkt durch Ansaugen des Produkts aus der besagten, das Produkt enthaltende Quelle zu ermöglichen, wobei der besagte Flakon **dadurch gekennzeichnet ist, dass** eine obere Trennwand (40) im Körper (1) mittels einem dichten Verbindungsmittel (41) integriert ist, die manuell zerstört werden kann, wo die besagte Entnahmevorrichtung (5) mit dem in der eingedrückten Position auf der besagten oberen Trennwand (40) gehaltenen Druckknopf (3) im Körper montiert wird, derart, dass das besagte dichte Verbindungsmittel (41) mit dem besagten Reservoir verbunden wird und kommunizieren kann.

2. Abgabeflakon gemäß Anspruch 1, **dadurch gekennzeichnet, dass** der Körper (1) einen verdünnten Bereich (41) umfasst, der unter der oberen Trennwand (40) angeordnet ist, um das dichte Verbindungsmittel zu bilden.

3. Abgabeflakon gemäß Anspruch 1 oder 2, **dadurch gekennzeichnet, dass** die obere Trennwand (40) von einer Schürze (47) eingefasst ist, wobei das dichte Verbindungsmittel (41) unter der besagten Schürze gebildet ist.

4. Abgabeflakon gemäß Anspruch 3, **dadurch gekennzeichnet, dass** die Schürze (47) eine äußere Umfangsfläche mit Riffeln (48) aufweist.

5. Abgabeflakon gemäß einem der vorstehenden Ansprüche 1 bis 4, **dadurch gekennzeichnet, dass** das dichte Verbindungsmittel (41) sich über den gesamten Umfang des Körpers (1) erstreckt, um die obere Trennwand (40) des besagten Körpers nach der Zerstörung des besagten Verbindungsmittels zu trennen.

6. Abgabeflakon gemäß einem der vorstehenden Ansprüche 1 bis 5, **dadurch gekennzeichnet, dass** der Körper (1) eine untere Öffnung (1 b) aufweist, in der das Füllventil (15) dicht montiert ist.

7. Abgabeflakon gemäß einem der vorstehenden Ansprüche 1 bis 6, **dadurch gekennzeichnet, dass** die Entnahmevorrichtung (5) ein Gehäuse (10), das mittels einer Buchse (42) im Körper (1) montiert ist, wo die besagte Buchse eine Bohrung (43) aufweist, in der das besagte Gehäuse befestigt ist, sowie eine äußere, dicht im Innern des besagten Körpers anliegende Krone (44) aufweist.

8. Abgabeflakon gemäß Anspruch 7, **dadurch gekennzeichnet, dass** das Innere des Körpers (1) eine radiale Rille (45) aufweist, auf der die äußere Krone (44) in axialem Anschlag ist.

9. Abgabeflakon gemäß Anspruch 7 oder 8, **dadurch gekennzeichnet, dass** das Innere des Körpers (1) mindestens eine axiale Rille (46) zur Entlüftung während der Montage der Buchse (42) im Körper (1) aufweist.

10. Abgabeflakon gemäß einem der vorstehenden Ansprüche 1 bis 9, **dadurch gekennzeichnet, dass** das Innere des Körpers (1) eine konisch nach oben zusammenlaufende Geometrie aufweist.

11. Abgabeflakon gemäß einem der vorstehenden Ansprüche 1 bis 10, **dadurch gekennzeichnet, dass** das Ventil (15) eine starre Haube (20) mit einem Innenaufnahmeraum (21) aufweist, in dem ein verformbares Ventil (22) montiert ist, wobei besagte Haube dicht im besagten Körper montiert ist, um den Innenaufnahmeraum (21) über einen oberhalb gelegenen Durchgang (23) in Kommunikation mit dem Reservoir (2) zu versetzen.

## Claims

1. Bottle for dispensing a fluid product comprising a body (1) wherein a container (2) for packaging said product is formed, said bottle further comprising an extraction device (5) of said packaged product which is tightly mounted in said body, said extraction device being equipped with a push button (3) which is provided with a dispensing orifice (4) of said product, said push button being reversibly movable along an actuation stroke of said device between an idle position and an engaged position wherein the dispensing orifice (4) is connected to the container (2) via said extraction device (5), said body being equipped with a filling valve (15) of said container which is arranged to enable the connection of a product source (16) with said container, the container (2) having a negative air pressure which is arranged to be able to carry out the filling of the container (2) with product by suction of the product contained in said source, said bottle being **characterised in that** an upper wall (40) is integrated in the body (1) by means of tight linking means (41) which can be manually destroyed, said extraction device (5) being mounted in said body with the push button (3) held in the engaged position on said upper wall (40) so as to connect the tight linking means (41) with said container.

2. Dispensing bottle according to claim 1, **characterised in that** the body (1) comprises a thinner area (41) which is arranged below the upper wall (40) to form the tight linking means.

3. Dispensing bottle according to claim 1 or 2, **characterised in that** the upper wall (40) is surrounded by a skirt (47), the tight linking means (41) being formed under said skirt.

4. Dispensing bottle according to claim 3, **characterised in that** the skirt (47) has an outer bearing surface provided with striations (48).

5. Dispensing bottle according to any one of claims 1 to 4, **characterised in that** the tight linking means (41) extend over the entire periphery of the body (1) so as to be able to separate the upper wall (40) from said body after said linking means have been destroyed.

6. Dispensing bottle according to any one of claims 1 to 5, **characterised in that** the body (1) has a lower opening (1b) wherein the filling valve (15) is tightly mounted.

7. Dispensing bottle according to any one of claims 1 to 6, **characterised in that** the extraction device (5) has a casing (10) which is mounted in the body (1) by means of a bush (42), said bush comprising a bore (43) wherein said casing is attached and an outer ring (44) tightly bearing on the inside of said body.

8. Dispensing bottle according to claim 7, **characterised in that** the inside of the body (1) has a radial groove (45) whereon the outer ring (44) is in axial abutment.

9. Dispensing bottle according to claim 7 or 8, **characterised in that** the inside of the body (1) has at least one axial venting groove (46) during the mounting of the bush (42) in the body (1).

10. Dispensing bottle according to any one of claims 1 to 9, **characterised in that** the inside of the body (1) has a conical geometry converging at the top.

11. Dispensing bottle according to any one of claims 1 to 10, **characterised in that** the valve (15) comprises a rigid cap (20) having an inner recess (21) wherein a deformable flap (22) is mounted, said cap being tightly mounted in said body in order to connect the inner recess (21) with the container (2) via a downstream passage (23).
